# FASCICULE DE BREVET EUROPEEN

(11) **EP 1 074 262 B1**
(45) Date de publication et mention de la délivrance du brevet: **20.04.2005**
(21) Numéro de dépôt: 00402210.9
(22) Date de dépôt: 02.08.2000
(51) Int. Cl.: A61K 35/80, A61P 17/00

(54) **Extrait d'algues Laminaria, procédé de préparation et compositions cosmétiques ou pharmaceutiques le contenant**
Laminaria Algenextrakt, Verfahren zur Herstellung und diese enthaltende kosmetische oder pharmazeutische Zusammensetzungen
Laminaria algae extract, method of preparation and cosmetic or pharmaceutical compositions containing the same

(30) Priorité: 03.08.1999 FR 9910076
(43) Date de publication de la demande: 07.02.2001
(73) Titulaire: SECMA BIOTECHNOLOGIES MARINES, 22260 Pontrieux (FR)
(72) Inventeur: Mekideche, Nicole, 22500 Paimpol (FR)
(74) Mandataire: Lhuillier, René

(56) Documents cités:
- PATENT ABSTRACTS OF JAPAN vol. 1997, no. 11, 28 novembre 1997 (1997-11-28) & JP 09 176034 A (LION CORP), 8 juillet 1997 (1997-07-08)
- GERALD BLUNDEN ET AL: "LYSINE BETAINE AND OTHER QUATERNARY AMMONIUM COMPOUNDS FROM BRITISH SPECIES OF THE LAMINARIALES" JOURNAL OF NATURAL PRODUCTS,XX,XX, vol. 45, no. 4, juillet 1982 (1982-07), pages 449-452-452, XP002099991 ISSN: 0163-3864
- WALDEMAR EICHENBERGER ET AL.: "BETAINE LIPIDS AND PHOSPHOLIPIDS IN BROWN ALGAE" PHYTOCHEMISTRY., vol. 34, no. 5, 1993, pages 1323-1333, XP000910787 PERGAMON PRESS., GB ISSN: 0031-9422

## Description

L'invention concerne un extrait d'algues enrichi en bétaïnes, son procédé d'extraction et son utilisation comme principe actif dans des compositions cosmétiques ou pharmaceutiques.

C'est un souci constant des fabricants de produits cosmétiques de proposer des compositions qui assurent une protection de la peau, principalement, contre les agressions du milieu extérieur dues notamment à la pollution, aux écarts de température, aux variations d'humidité et, dans des circonstances plus particulières, au contact prolongé avec l'eau de mer et à l'exposition au soleil.

Divers principes actifs sont recherchés, de préférence dans des sources naturelles, végétales ou animales. En effet, on trouve dans la nature plusieurs molécules qui participent à la défense des cellules contre diverses conditions de stress. Parmi celles-ci, les bétaïnes sont connues pour leur rôle dans le maintien de l'homéostasie et, plus particulièrement, dans la protection du milieu intracellulaire contre le stress osmotique (voir notamment Petronini et al. Biochem. J. 282, 1992, 69-73 et Sutherland et al. J. Bacteriol. 168, 1986, 805-814).

Ces molécules se retrouvent aussi bien dans le monde animal que végétal, ou dans les bactéries et le plancton.

Elles sont abondantes dans les algues qui sont soumises à des variations de pression osmotique, en particulier dans les algues qui sont alternativement soumises au contact des vagues et à l'émersion.

C'est pourquoi la Demanderesse a tiré parti de l'abondance des algues brunes de la famille des Phéophycées sur les côtes de Bretagne pour y rechercher des molécules potentiellement intéressantes, notamment pour l'industrie cosmétique.

Elle s'est plus particulièrement attachée à préparer des extraits de l'algue Laminaria ochroleuca et à enrichir lesdits extraits en substances de la famille des bétaïnes, par un procédé facile à mettre en oeuvre à l'échelle industrielle.

Le procédé selon l'invention comprend :
a) - la récolte des algues fraîches,
b) - leur lavage,
c) - un broyage et un microbroyage,
d) - l'élimination des débris cellulaires par centrifugation,
e) - un fractionnement par précipitation à l'acide, de préférence à l'HCl, et adsorption sur charbon activé,
f) - une filtration contrôlée par dosage de la glycine-bétaïne en HPLC, et qui est arrêtée quand le filtrat atteint une concentration en glycine-bétaïne de 12,5 %. Ladite filtration est de préférence une filtration tangentielle avec un seuil de coupure à 1 000 D.

L'extrait ainsi obtenu, standardisé pour son contenu en glycine-bétaïne, qui en représente au moins 10 %, sera appelé Laminaïne, en raison de sa source de matière première, la laminaire.

Outre les propriétés connues des bétaïnes comme osmoprotecteur, la Demanderesse a mis en évidence un effet antiradicalaire et un effet stimulateur sur la néosynthèse de constituants de membranes cellulaires et de la matrice extracellulaire (protéoglycannes et glycosaminoglycannes) et elle a tiré parti de ces propriétés inattendues pour proposer l'utilisation de l'extrait d'algues selon l'invention comme principe actif, notamment dans des compositions à usage cosmétique.

L'ensemble des propriétés de l'extrait d'algues mises en évidence par la Demanderesse et qui apparaîtront dans les exemples qui suivent permettent en effet d'envisager l'utilisation de cet extrait notamment pour améliorer l'état de la peau et sa résistance aux diverses conditions de stress qui provoquent la libération de radicaux libres et/ou qui ralentissent la régénération de la peau et qui en accélèrent le vieillissement.

Ainsi l'invention concerne l'utilisation de l'extrait d'algues décrit plus haut, dans des compositions cosmétiques ou pharmaceutiques, plus particulièrement comme principe actif osmoprotecteur, antiradicalaire et contre le vieillissement cutané.

Ce principe actif peut être utilisé pour être incorporé dans toute composition cosmétique ou pharmaceutique de type crème, gel, émulsion, lait.

L'invention concerne donc également lesdites compositions cosmétiques ou pharmaceutiques, comprenant de 1 à 40 % d'extrait d'algues tel que décrit plus haut.

Le même principe actif pourrait être extrait à partir d'autres végétaux, par un procédé d'extraction similaire au procédé décrit ici.

L'objet de la présente invention s'étend également à l'utilisation dudit principe actif extrait de n'importe quelle plante qui en contient, en tant que principe actif antiradicalaire et/ou comme traitement du vieillissement de la peau.

Les exemples suivants illustrent l'invention sans en limiter la portée.

### Exemple 1 : Procédé d'extraction.

L'algue Laminaria ochroleuca est récoltée entre mai et septembre, sur les côtes nord de la Bretagne.

Les algues récoltées sont transportées en container, dans de l'eau de mer. Elles sont traitées dès leur arrivée sur le site de production.

Les algues sont lavées deux fois à l'eau déminéralisée puis à l'eau additionnée d'une solution stérilisante.

Elles sont broyées dans un broyeur à lames de type Urschell® et réduites en particules de taille comprise entre 100 et 200 µm.

Le broyat est mis en suspension dans de l'eau déminéralisée glycolée (à 50 %), à raison de 430 g de broyat dans 1 litre de solution.

Un broyage plus fin, destiné à libérer le contenu cellulaire est réalisé à l'aide d'un homogénéisateur à lames de type Ultra Turax®, à température ambiante, pendant trois heures.

Le broyat est centrifugé à 16 000 g pendant 45 min pour éliminer les débris cellulaires. Le surnageant est récupéré et concentré sous vide à un tiers du volume initial.

Il est ensuite dilué 20 fois dans l'eau distillée puis additionné d'HCl à 35 % pour amener son pH à 1,0, de 4 % de charbon actif, et il est soumis à une agitation magnétique pendant 30 minutes.

L'addition d'acide permet de précipiter de nombreuses substances indésirables (en particulier des protéines) et le charbon adsorbe diverses substances organiques (de type phénols...).

Le surnageant est ensuite enrichi en bétaïnes par filtration tangentielle avec un seuil de coupure de 1 000 D. Le filtrat clair est récolté et analysé par chromatographie HPLC avec détecteur UV, selon la méthode de Zamarreno (J. Agricultural and Food Chemistry, 45,3 ; 774-776).

On utilise comme standard de la glycine-bétaïne purifiée (Sigma), en solution dans l'eau distillée à 0,2 , 0,4 , 0,8 et 2 % en poids, dilué vingt fois, pour établir une courbe de calibrage.

L'échantillon d'extrait d'algue est dosé par comparaison de la surface du pic de chromatographie avec la courbe standard.

On arrête le procédé d'enrichissement quand la concentration de glycine-bétaïne atteint 12, 5 % ± 2 ,5 %. L'extrait ainsi obtenu sera appelé "Laminaïne" dans les exemples suivants.

### Exemple 2 : Mise en évidence d'un effet antiradicalaire.

La mise en évidence d'un effet antiradicalaire de la Laminaïne a été obtenue par dosage du malondialdéhyde dans des fibroblastes de derme humain mis en culture à partir d'une biopsie de peau. Les tests sont réalisés sur des cultures entre le 2ème et le 4ème passage.

Dosage du malondialdéhyde (MDA) : Indice de lipopéroxydation.

Les fibroblastes ont été répartis dans des boîtes multipuits (24 puits) à raison de 10⁵ cellules par puits dans 1 ml de milieu de culture RPMI 1640 supplémenté par 10 % de sérum de veau foetal, 10 mM de L-glutamine et 80 µg/ml de gentamicyne. Elles ont ensuite été maintenues pendant 24 heures en étuve à CO₂.

La Laminaïne a été répartie aux différentes concentrations (pur, 1/2, 1/5 et 1/10) dans les plaques multipuits à raison de 3 puits par dose. Parallèlement, différents contrôles ont été réalisés :
- 3 puits ont seulement reçu le solvant ;
- 3 puits ont reçu la SOD (Superoxyde Dimustase) et la catalase (témoin négatif) ;
- 3 puits ont reçu le complexe xanthine-hypoxanthine (contrôle de l'efficacité des enzymes de protection (SOD + catalase) ;
- 12 puits ont reçu les 4 dilutions de la Laminaïne en plus de la xanthine-hypoxanthine ;
- 12 puits ont reçu les 4 dilutions de la Laminaïne.

### Extraction du malondialdéhyde.

Après trypsinisation et centrifugation des cellules, les culots cellulaires ont été remis en suspension dans :
- 250 µl de tampon Tris 50 mM, pH 8 contenant NaCl 0,1 M ;
- EDTA 20 mM
- 25 µl de SDS à 7 %
- 300 µl de HCl (0,1 N)
- 38 µl d'acide phosphotungstique à 1 % dans l'eau
- 300 µl d'acide thiobarbiturique à 0,67 % dans l'eau.

Après 1 heure d'incubation dans l'obscurité à 50°C et un refroidissement dans l'eau glacée, 300 µl de n-butanol ont été ajoutés dans chaque tube. Ceux-ci ont été centrifugés à 10 000 g à 0°C pendant 10 minutes. La phase supérieure a été récupérée pour le dosage du MDA.

### Dosage du malondialdéhyde.

Le MDA a été dosé par mesure de la fluorescence après séparation du complexe MDA-TBA par HPLC.
- Pompe Bischoff Model 2.200
- Injecteur automatique Alcott Model autosampler
- Colonne Ultrasep C18 (30 cm x 0, 18 cm) 6 µm de porosité
- Détecteur de fluorescence, jasco 821-F1

La détection de la fluorescence a été effectuée avec une excitation à 515 nm et une émission à 553 nm. L'éluent utilisé est composé de méthanol : eau, 40 : 60 (v/v) dont le pH 8,3 a été ajusté avec du KOH 1M.

La quantification a été faite par rapport à des standards traités comme les échantillons (0,125 ; 0,25 ; 0,5 ; 1 µM) à l'aide d'un logiciel informatique ICS (Pic 3). (Instrumentation, Consommable Service).

### Dosage des protéines.

Le dosage des protéines a été réalisé selon la méthode de BRADFORD. L'augmentation de l'absorbance à 595 nm est proportionnelle à la concentration des protéines déterminées à l'aide d'un spectrophotomètre UNICAM 8625.

### Résultats - Dosage du malondialdéhyde (MDA) dans l'homogénat cellulaire.

### 1 - lipopéroxydation physiologique.

| Laminaïne | MDA (µM/mg protéines) | % de diminution du MDA |
|---|---|---|
| Témoin 0 | 618 ± 58 | - |
| Pur | 558 ± 19 | - 5 |
| Dilution 1/2 | 556 ± 76 | - 10 |
| Dilution 1/5 | 481 ± 65 | - 22 |
| Dilution 1/10 | 467 ± 69 | - 24 |

On observe une protection significative contre la lipopéroxydation physiologique en présence de Laminaïne, surtout aux dilutions 1/5 et 1/10.

### 2 - Lipopéroxydation provoquée.

Pour ce dosage, il n'a été retenu que les dilutions à 1/5 et 1/10 compte tenu de la diminution non significative du MDA physiologique avec la Laminaïne pure et diluée à 1/2.

| Réactifs : | MDA (µ M/mg protéines) | % de diminution du MDA |
|---|---|---|
| Témoin | 656 ± 39 | - |
| SOD-Cat | 418 ± 49 | - 36 |
| Xant-Hypox | 1156 ± 174 | + 76 |
| SOD-Cat-Xant-Hypox | 806 ± 71 | - 30 |
| 1/5-Xant-Hypox | 804 ± 24 | - 30 |
| 1/10-Xant-Hypox | 460 ± 25 | - 60 |

On observe une protection importante de la Laminaïne contre la lipopéroxydation provoquée par le système générateur de radicaux libres Xanthine/Hypoxanthine, aux dilutions 1/5 et 1/10, au moins égale à celle apportée par les enzymes protectrices connues, SOD-Catalase.

Dans les conditions expérimentales retenues, la Laminaïne présente donc une activité antiradicalaire significative vis-à-vis des fibroblastes humains en culture après 24 heures de contact.

### Exemple 3 - Mise en évidence d'une stimulation de la synthèse des protéoglycannes et glycosaminoglycannes par les fibroblastes en culture.

Les fibroblastes mis en culture à partir de biopsie de peau humaine et multipliés par 2 à 4 passages ont été répartis dans des boîtes de 25 cm² à raison de 10⁵/ml dans 10 ml de milieu de culture RPMI 1640 supplémenté par 10 % de sérum de veau foetal, 10 mM de L-glutamine et 80 µg/ml de gentamicyne. Elles ont ensuite été maintenues 24 heures en étuve à CO₂.

La Laminaïne a été répartie aux différentes concentrations (dilution 1/2, 1/5 et 1/10) dans les boîtes à raison de 3 boites par dose. Parallèlement, 3 boîtes ont reçu seulement de l'eau et ont servi de témoin.

Afin d'étudier la capacité des cellules, préalablement traitées par la Laminaïne, à incorporer le précurseur radioactif, la technique de pulse a été adoptée. Le précurseur radioactif ([3H]-Glucosamine) a été ajouté aux cultures 18 heures avant la récupération des cellules. Le temps de contact des cellules avec le produit a été de 24 heures à 37°C.

Après avoir éliminé le milieu par aspiration, les cellules ont été lavées 2 fois avec du milieu sans sérum pour éliminer la radioactivité non incorporée, puis détachées du support par grattage de la surface de la culture. Les cellules ont été lavées une nouvelle fois avec du milieu puis centrifugées à 600 g pendant 5 minutes. Sur ce culot cellulaire, on réalise :
- le dosage des protéoglycannes par FPLC (Fast protein liquid Chromatography)
- la néosynthèse des glycosaminoglycannes totaux (GAGs)
- le dosage des protéines totales
- la caractérisation des glycosaminoglycannes par digestion sélective

### 1 - Extraction.

### Protéoglycannes membranaires.

Une première fraction du culot a été resuspendu dans NaCl 1 M contenant la désoxyribonucléase (50 U/ml) et des inhibiteurs de protéases. L'homogénat a ensuite été incubé à 4°C pendant 2 heures. Une centrifugation pendant 30 minutes à 12 000 g a permis d'obtenir le premier homogénat contenant les protéoglycannes péri-membranaires. Le culot a servi à extraire les protéoglycannes des 2 autres compartiments (Cl).

### Protéoglycannes transmembranaires.

Le culot Cl obtenu à partir de la première étape d'extraction a été resuspendu dans l'azide de sodium 0,1 % contenant du désoxycholate de sodium 4 %, soniqué à 75 mv pendant 20 secondes puis incubé 2 heures à 4°C. Une centrifugation pendant 30 minutes à 12 000 g a permis d'obtenir le deuxième surnageant contenant les protéoglycannes transmembranaires. Le culot a servi à extraire les protéoglycannes du compartiment matriciel (C2).

### Protéoglycannes matriciels.

Le culot C2 a été lavé trois fois avec l'azide de sodium 0,1 % puis a été remis en suspension sous agitation dans un tampon acétate de sodium 50 mM contenant la guanidine HCl 4 M et du triton X-100 0,1% ainsi que des inhibiteurs de protéases.

Une centrifugation pendant 30 minutes à 12 000 g a permis d'obtenir le troisième surnageant contenant les protéoglycannes matriciels.

### 2 - Purification par FPLC.

Préalablement à la purification par FPLC, chacun des 3 surnageants a été précipité une nuit à 4°C dans 3 volumes d'éthanol absolu, puis centrifugé à 12 000 g pendant 30 minutes. Les culots obtenus ont été resuspendus dans un tampon tris-HCl 50 mM pH 7,4.

Le même protocole d'analyse a été réalisé pour les 3 échantillons.

La chromatographie échangeuse d'anions a été utilisée. En effet, elle est favorisée par la grande densité de charges négatives apportées aux protéoglycannes par leurs chaînes de glycosaminoglycannes.

Chaque culot a été resuspendu dans 250 ml du tampon Tris-HCl 50 mM pH 7,4. Le gel Sepharose® CL-6B-DEAE (Pharmacia) a été coulé dans une colonne K 10/40 (Pharmacia). Ce gel échangeur d'anions a une grande résolution et un bon rendement. 100 µl de chaque échantillon ont été injectés, l'élution est suivie par détection au spectrofluorimètre à longueur d'onde 280 nm. Le pic contenant les protéoglycannes est élué à 1 M de NaCl.

### 3 - Dosage.

Le dosage de la radioactivité est réalisé à la sortie d'HPLC avec un compteur Packard (Flo-one).

### 4 - Identification par dégradation sélective.

Pour déterminer la nature des GAGs présents dans les fractions, différentes réactions de dégradation ont été réalisées.

### Digestion par la chondroïtinase AC.

La chondroïtinase AC dépolymérise les acides glucuroniques dégradant ainsi la chondroïtine sulfate.

Un aliquot du lyophilisat de GAGs a été incubé avec la chondroïtinase AC (0,2 U/ml) dans un tampon Tris-HCl pH 8 pendant 1 heure à 37°C. La réaction est stoppée par congélation à -20°C.

### Digestion par la chondroïtinase ABC.

La chondroïtinase ABC dépolymérise les acides glucuroniques et iduroniques dégradant ainsi la chondroïtine sulfate et le dermatane sulfate.

Un deuxième aliquot du lyophilisat de GAGs a été incubé avec la chondroïtinase ABC (0,5 U/ml) dans le même tampon Tris-HCl pH 8 pendant 1 heure à 37°C. La réaction est stoppée à -20°C.

### Digestion par l'acide nitreux.

La solution d'acide nitreux a été préparée en mélangeant le nitrate de sodium (0,148 M) et l'acide acétique (3,6 M).

L'acide nitreux coupe les liaisons glucosidiques des liaisons glucosamines ayant un groupement aminosulfonate en position 2 ; il est, de ce fait, spécifique de l'héparine et de l'héparane sulfate.

La réaction a eu lieu à température ambiante pendant 80 minutes ; elle a été stoppée par addition de sulfonate d'ammonium 1M. L'hydrolysat a été évaporée à sec puis repris dans du Tris-HCl 50 mM pour analyse.

### Séparation des GAGs sur gel CL.6B (Pharmacia).

Ce gel est adapté à l'étude des molécules de faible poids moléculaire. Il a été équilibré par un tampon Tris-HCl 50 mM, pH 7,4 4 puis coulé dans une colonne K 10/40 (Pharmacia). L'élution a été réalisée par le même tampon contenant du NaCl 0,35 M. Des fractions de 1 ml ont été recueillies puis comptées après addition de 5 ml de liquide scintillant.

### Résultats des dosages de radioactivité : effet sur la néosynthèse des protéoglycannes.

- Protéoglycannes péri-membranaires.

| Laminaïne | cpm | % d'incorporation |
|---|---|---|
| Témoin 0 | 155 ± 32 | - |
| Dilution 1/5 | 208 ± 25 | 34 |
| Dilution 1/10 | 215 ± 12 | 38 |

- Protéoglycannes membranaires.

| Laminaïne | cpm | % d'incorporation |
|---|---|---|
| Témoin 0 | 170 ± 25 | - |
| Dilution 1/5 | 253 ± 12 | 48 |
| Dilution 1/10 | 285 ± 17 | 67 |

- Protéoglycannes matriciels.

| Laminaïne | cpm | % d'incorporation |
|---|---|---|
| Témoin 0 | 135 ± 25 | - |
| Dilution 1/5 | 170 ± 43 | 25 |
| Dilution 1/10 | 154 ± 19 | 14 |

On observe donc une stimulation significative de la néosynthèse de protéoglycannes en présence de Laminaïne.

### Résultats de caractérisation des glycosaminoglycannes néosynthétisés.

- Néosynthèse de dermatane et héparane sulfate (DS et HS).

| Laminaïne | cpm | % d'incorporation |
|---|---|---|
| Témoin 0 | 256 ± 24 | - |
| Dilution 1/5 | 263 ± 43 | 0 |
| Dilution 1/10 | 308 ± 21 | 20 |

- Néosynthèse de dermatane et chondroïtine sulfate (DS et CS) .

| Laminaïne | cpm | % d'incorporation |
|---|---|---|
| Témoin 0 | 538 ± 85 | - |
| Dilution 1/5 | 558 ± 73 | - |
| Dilution 1/10 | 498 ± 103 | - |

- Néosynthèse d'héparane sulfate (HS).

| Laminaïne | cpm | % d'incorporation |
|---|---|---|
| Témoin 0 | 304 ± 23 | - |
| Dilution 1/5 | 345 ± 65 | - |
| Dilution 1/10 | 398 ± 38* | 30 |

En conclusion, la Laminaïne diluée à 1/5 et 1/10 présente une activité significative sur la néosynthèse des constituants matriciels des fibroblastes humains en culture.

En effet, les résultats obtenus montrent une stimulation significative de la néosynthèse des protéoglycannes membranaires (67 %), péri-membranaires (environ 34 %) et matriciels (25 % à la dilution 1/5).

Parallèlement, les dosages des couples DS/HS, DS/CS et HS révèlent une stimulation de la production d'héparane sulfate (30 % à la dilution au 1/10), qui est présent surtout au niveau de la membrane cellulaire. La présence de PGs au niveau cellulaire a une incidence importante sur le métabolisme à la fois des cellules et de la matrice extracellulaire. Ces PGs, de par leur localisation, pourraient établir divers types d'interactions cellule-ligand, cellule-cellule, cellule-matrice.

### Exemple 4 - Mise en évidence d'une stimulation de la synthèse du collagène par les fibroblastes en culture.

Les fibroblastes sont mis en culture dans les mêmes conditions que dans l'exemple 3.

### Pré-incubation des cellules.

La pré-incubation a pour but de synchroniser les cellules en phase G1 ou G0 du cycle cellulaire en ralentissant leur activité par diminution des facteurs sériques. Après avoir atteint le stade de confluence, le milieu est éliminé et remplacé par du milieu RPMI additionné de 0,5 % de SVF. La période de pré-incubation se poursuit pendant 24 heures.

### Incubation des cellules.

La pré-incubation terminée, le milieu est éliminé et la couche cellulaire est rincée par du milieu RPMI sans SVF pour éliminer toute trace de constituants sériques. Les incubations sont réalisées avec du milieu de culture additionné soit de vitamine C à la concentration de 0,28 mmol/l, soit de la Laminaïne à différentes dilutions.

La concentration de vitamine C utilisée est optimale pour l'activation des enzymes d'hydroxylation, ainsi que pour la sécrétion du collagène. On ajoute également du β-aminopropionitrile à la concentration de 0,2 mmole/l, pour empêcher le dépôt du collagène néosynthétisé sous forme de fibrilles dans le milieu de culture, par inhibition de l'enzyme lysyl-oxydase.

A la fin de la période d'incubation (24 heures), le milieu de culture est récupéré et la couche cellulaire est rincée.

Dosage de l'hydroxyproline par HPLC.

### - Principe de la méthode.

Les acides aminés sont dérivés par l'acide ophtaldéhyde (l'OPA) éliminant ainsi leur interférence. L'hydroxyproline et la proline sont alors dérivés par le NBD-Cl par couplage des groupements aminés au NBD-C1. Le NBD-Hyp est séparé et identifié par HPLC en phase inverse. Pour la mise au point de la séparation des dérivés d'acides aminés, un couplage au NBD-Cl d'un standard contenant l'hydroxyproline a tout d'abord été réalisé.

### - Matériels.

L'hydroxyproline est dosée par mesure de la fluorescence après séparation de l'hydroxyproline par HPLC en phase inverse :
- Pompe Bischoff Model 2.200
- Injecteur automatique Alcott Model 788 autosampler
- Colonne Ultrasep C18 (30 cm x 0,18 cm) 6 µm de porosité
- Détecteur de fluorescence, Jasco 821-FI
- Conditions chromatographiques.

La phase mobile est constituée d'un mélange d'acétonitrile/tampon phosphate de sodium 0,1 mol/l, pH 7,2 (9:91 v/v), le débit est réglé à 1 ml/minute, l'élution est réalisée en mode statique et le cycle est de 10 minutes. La phase mobile est préalablement filtrée puis dégazée avant utilisation.

### - Préparation des réactifs.

NBD-Cl = 25 mmol sont dissous dans le méthanol
OPA = 150 mmol/l dans du méthanol
Tampon = 0,4 mmol/l, le pH est ajusté à 7,2.

Solution aqueuse d'hydroxyproline (50 mg/l), puis diluée pour obtenir des concentrations allant de 0,5 à 40 mg/l.

### - Gamme étalon.

10 µl de solution étalon à différentes concentrations sont mélangées avec 10 µl du tampon (pH 9). Après addition de 5 µl d'OPA et agitation, les tubes sont gardés 5 minutes à température ambiante puis 10 µl de la solution NBD-Cl sont ajoutés. Le dérivât se fait à 60°C pendant 3 minutes à l'abri de la lumière. Les tubes sont ensuite mis dans la glace. La coloration obtenue est orange et stable pendant au moins 3 heures à l'abri de la lumière. 50 µl de ce mélange sont ensuite injectés. La courbe d'étalonnage est linéaire.

Les échantillons de milieu de culture sont traités de la même façon et l'essai est renouvelé trois fois.

### • Résultats.

La séparation et l'identification de l'hydroxyproline ont été réalisés par HPLC en phase inverse. Les pics de fluorescence, après intégration, ont permis de calculer la concentration de l'hydroxyproline dans le milieu de culture.

| | Concentration d'hydroxyproline mg/ml | % d'augmentation |
|---|---|---|
| Témoin 0 | 3,67 ± 1,18 | - |
| Témoin + Vit C | 5,25 ± 1,31 | 43 |
| Dilution 1/5 | 9,72 ± 1,32 | 164 |
| Dilution 1/10 | 8,64 ± 0,98 | 135 |

On observe que la Laminaïne diluée à 1/5 et 1/10 induit, sur les fibroblastes humains à confluence, une néosynthèse accrue du collagène plus importante que celle induite par la vitamine C (43 %) utilisée en concentration optimale pour la synthèse du collagène et l'activation des enzymes d'hydroxylation.

## Revendications

1. Procédé d'obtention d'un extrait d'algues enrichi en bétaïnes par élimination de débris cellulaires, **caractérisé en ce qu'**il coporte les étapes suivantes :
a) - récolte d'algues de la famille des Phéophycées,
b) - lavage des algues,
c) - broyage et microbroyage,
d) - élimination des débris cellulaires par centrifugation,
e) - fractionnement par précipitation à l'acide et par adsorption sur du charbon activé,
f) - filtration contrôlée par dosage de la glycine-bétaïne en HPLC.

2. Procédé selon la revendication 1, dans lequel les algues sont des Laminaria ochroleuca.

3. Procédé selon l'une des revendications 1 et 2, dans lequel l'acide est HCl.

4. Procédé selon l'une des revendications 1 à 3, dans lequel la filtration est tangentielle avec un seuil de coupure de 1 000 D.

5. Extrait d'algues enrichi en bétaïnes susceptible d'être obtenu par le procédé selon l'une quelconque des revendications 1 à 4.

6. Extrait selon la revendication 5, contenant au moins 10% de glycine-bétaïne.

7. Extrait d'algues selon l'une des revendications 5 et 6 pour usage thérapeutique.

8. Utilisation de l'extrait d'algues selon l'une des revendications 5 et 6 comme principe actif dans des compositions à usage cosmétique pour l'un au moins des effets du groupe constitué par l'effet osmoprotecteur, l'effet anti-radicalaire et l'effet contre le vieillissement cutané.

9. Composition cosmétique ou pharmaceutique de type crème, gel, émulsion, lait, **caractérisée en ce qu'**elle comprend de 1 à 40% d'extrait d'algues selon l'une des revendications 5 et 6.

## Patentansprüche

1. Verfahren zur Gewinnung eines an Betainen angereicherten Algenextrakts durch Eliminierung von Zellbruchstücken, **dadurch gekennzeichnet, dass** es die folgenden Stufen umfasst;
a) das Ernten von Algen aus der Familie der Pheophyceae,
b) das Waschen der Algen,
c) das Zerkleinern und Feinstmahlen derselben,
d) das Eliminieren von Zellbruchstücken durch Zentrifugieren,
e) das Fraktionieren durch Ausfällung mittels Säure und durch Adsorption an Aktivkohle und
f) das kontrollierte Filtrieren durch Zudosierung des Glycinbetains bei der HPLC-Chromatographie.

2. Verfahren nach Anspruch 1, bei dem es sich bei den Algen um solche aus der Familie Laminaria ochroleuca handelt.

3. Verfahren nach einem der Ansprüche 1 bis 2, bei dem die Säure HCl ist.

4. Verfahren nach einem der Ansprüche 1 bis 3, bei dem die Filtration eine Tangential-Filtration mit einem Fraktionier-Schwellenwert von 1000 D ist.

5. Algenextrakt, der an Betainen angereichert ist, wie er nach dem Verfahren nach einem der Ansprüche 1 bis 4 erhältlich ist.

6. Algenextrakt nach Anspruch 5, der mindestens 10 % Glycinbetain enthält.

7. Algenextrakt nach einem der Ansprüche 5 und 6 für die therapeutische Verwendung.

8. Verwendung des Algenextrakts nach einem der Ansprüche 5 und 6 als Wirkstoff in Zusammensetzungen für die kosmetische Verwendung wegen mindestens einer der folgenden Wirkungen: Osmoschutzwirkung, Antiradikal-Wirkung und Wirkung gegen Hautalterung.

9. Kosmetische Zusammensetzung oder pharmazeutische Zusammensetzung vom Creme-, Gel-, Emulsions- und Milch-Typ, **dadurch gekennzeichnet, dass** sie 1 bis 40 % Algenextrakt nach einem der Ansprüche 5 und 6 enthält.

## Claims

1. A method for obtaining an extract of algae enriched in betaines by elimination of cell debris, **characterized in that** it comprises the following steps:
a) - harvesting algae of the family Phaeophyceae,
b) - washing the algae,
c) - grinding and microgrinding,
d) - elimination of the cell debris by centrifugation,
e) - fractionation by precipitation with acid and by adsorption on activated charcoal,
f) - filtration controlled by assaying the glycine-betaine by HPLC.

2. A method according to claim 1, in which the algae are Laminaria ochroleuca.

3. A method according to either of claims 1 and 2, in which the acid is HCl.

4. A method according to one of claims 1 to 3, in which the filtration is tangential with a cut-off threshold of 1000 D.

5. An extract of algae enriched in betaines that can be obtained by means of the method according to any one of claims 1 to 4.

6. An extract according to claim 5, containing at least 10% of glycine-betaine.

7. An extract of algae according to either of claims 5 and 6, for therapeutic use.

8. Use of the extract of algae according to either of claims 5 and 6, as an active principle in compositions for cosmetic use for at least one of the effects of the group consisting of the osmoprotective effect, the free-radical scavenger effect and the effect against skin aging.

9. A cosmetic or pharmaceutical composition of the cream, gel, emulsion or milk type, **characterized in that** it comprises from 1 to 40% of extract of algae according to either of claims 5 and 6.
